(19) **Europäisches Patentamt**
**European Patent Office**
**Office européen des brevets**

(11) Numéro de publication : **0 344 040 B1**

(12) **FASCICULE DE BREVET EUROPEEN**

(45) Date de publication du fascicule du brevet :
**01.07.92 Bulletin 92/27**

(51) Int. Cl.$^5$ : **A61K 9/50,** A61K 47/00

(21) Numéro de dépôt : **89401317.6**

(22) Date de dépôt : **11.05.89**

(54) **Vecteur particulaire utile notamment pour le transport de molécules à activité biologique et procédé pour sa préparation.**

(30) Priorité : **27.05.88 FR 8807110**

(43) Date de publication de la demande :
**29.11.89 Bulletin 89/48**

(45) Mention de la délivrance du brevet :
**01.07.92 Bulletin 92/27**

(84) Etats contractants désignés :
**AT BE CH DE ES FR GB GR IT LI LU NL SE**

(56) Documents cités :
**EP-A- 0 240 424**
**GB-A- 2 185 397**

(72) Inventeur : **Samain, Daniel**
**16 Allée Henri Sellier**
**F-31400 Toulouse (FR)**
Inventeur : **Bec, Jean-Louis**
**137 Résidence des Côteaux**
**F-31520 Ramonville St. Agne (FR)**
Inventeur : **Cohen, Edith**
**56, Avenue de Gavarnie**
**F-31240 L'Union (FR)**
Inventeur : **Nguyen, Frédérique**
**30 Chemin des Maraîchers Bat. C Apt 81**
**F-31400 Toulouse (FR)**
Inventeur : **Peyrot, Marianne**
**74, Chemin des Clotasses**
**F-31400 Toulouse (FR)**

(74) Mandataire : **Warcoin, Jacques et al**
**Cabinet Régimbeau 26, avenue Kléber**
**F-75116 Paris (FR)**

(73) Titulaire : **CENTRE NATIONAL DE LA RECHERCHE SCIENTIFIQUE (CNRS)**
**15, Quai Anatole France**
**F-75700 Paris Cedex 07 (FR)**

**EP 0 344 040 B1**

## Description

La présente invention concerne un nouveau vecteur particulaire utile notamment pour le transport de molécules à activités biologiques.

Parmi les procédés utilisés pour faire pénétrer et réagir un composé (principe actif) à l'intérieur d'un système biologique ou biochimique il est parfois intéressant d'utiliser un vecteur particulaire dans lequel le produit actif est encapsulé.

C'est le cas notamment :

– Des composés ayant à l'état libre une durée de vie trop faible dans l'organisme ou dans des systèmes biologiques ou biochimiques. Le vecteur permet alors de le protéger et de lui assurer une diffusion constante.

– Des composés qui doivent agir sur un type de cellules particulier ou être présentés d'une certaine manière. C'est le cas des produits anticancéreux et des antigènes. Le vecteur peut alors assurer la présentation et le ciblage vers la cible choisie.

– Des composés qui ne sont pas résorbés naturellement au niveau des muqueuses : intestin, système ORL, vagin ou de la peau. Le vecteur sert alors d'enrobage pour faciliter le passage à travers la barrière.

Un tel vecteur particulaire doit satisfaire à un certain nombre d'exigences touchant notamment : à sa charge utile, sa stabilité, sa biométabolisation et ses possibilités de diffusion des principes actifs. Pour être intéressant, il doit, en outre, par ses propriétés, tendre à se rapprocher le plus possible des systèmes naturels de transport de molécules dans l'organisme ou rencontrés dans les milieux naturels, ceci afin de ne pas perturber l'équilibre physiologique et pouvoir éventuellement profiter des avantages liés aux processus vitaux naturels.

Parmi les systèmes de transport naturels que les vecteurs peuvent tenter d'imiter, les plus intéressants à étudier sont les lipoprotéines.

Les lipoprotéines présentent toutes le même type d'organisation structurale avec un noyau lipidique interne entouré de phospholipides. Une apolipoprotéine est de plus ancrée dans la couche phospholipidique et assure le guidage de la lipoprotéine. Il existe différentes sortes de lipoprotéines qui diffèrent par la taille, la nature des lipides transportés et la structure de l'apolipoprotéine. Les chylomicrons, par exemple, sont des lipoprotéines relativement grosses ($1\mu$m) et sont chargées de transporter les graisses provenant de la digestion. Les lipoprotéines basse densité ou LDL sont beaucoup plus petites (20-25 nm) et sont chargées de transporter le chlolestérol. La petite taille des LDL leur permet de diffuser à l'intérieur des tissus.

Le vecteur synthétique le plus proche de ces vecteurs naturels est le liposome qui est constitué d'une bicouche phospholipidique entourant une vacuole aqueuse. Les liposomes ont fait l'objet de nombreux travaux pour développer leur utilisation en tant que vecteurs de principes actifs.

Ils présentent cependant un certain nombre d'inconvénients qui ont empêché leur application dans l'industrie pharmaceutique. Ce sont notamment leur fragilité, leur hétérogénéité, leur faible capacité de transport et les difficultés posées par leur production à l'échelle industrielle.

Il est donc souhaitable de disposer d'un vecteur proche structurellement des vecteurs naturels mais ne présentant pas les inconvénients rencontrés avec les liposomes.

L'invention en cause concerne des vecteurs particulaires de nature phospholipide-glycolipide-polysaccharidique encore appelées Bio Vecteurs Supra Moléculaires (BVSM) qui permettent le transport de molécules d'origine naturelle, synthétique, hémisynthétique ou recombinante au sein de systèmes biologiques et biochimiques divers : eucaryotes, ou procaryotes, pour des réactions enzymatiques, chimiques, immunologiques, des tests de diagnostics, ou encore pour des fermentations.

Bien que des systèmes particulaires de transport de substances actives aient déjà été décrits, aucun d'entre eux ne présente la structure multicouche particulière des vecteurs particulaires selon l'invention qui permet l'obtention d'entités de taille définie, modulables, très stables, lyophilisables, stérilisables, entièrement métabolisables, et dont la structure et la surface présentent une grande similitude avec celles des vecteurs naturels.

Plus particulièrement, la présente invention concerne un vecteur particulaire caractérisé en ce qu'il comporte :

– un noyau hydrophile non liquide, essentiellement un polymère hydrophile, polysaccharide par exemple, réticulé,

– une première couche ou couronne de nature lipidique généralement liée au noyau par des liaisons covalentes et,

– une seconde couche ou feuillet de composés amphiphiles par exemple des phopholipides, des détergents biocompatibles ou des tensioactifs capables de s'organiser en micelles, cette couche étant en général stabilisée sur la première par les interactions hydrophobes.

Le noyau hydrophile, notamment polysaccharidique peut être obtenu par différentes méthodes, en particulier lorsqu'il s'agit d'un polysaccharide, on utilisera de préférence un polysaccharide biodégradable qu'il soit linéaire ou ramifié, par exemple des polysaccharides naturels ou non de type dextrane, amidon, cellulose, ou les dérivés de ces composés, en particulier les dérivés portant des charges ioniques positives ou négatives.

Le poids moléculaire de ces polysaccharides peut varier très largement de 50.000 à plusieurs (presque 10) millions de Daltons. Les procédés de réticulation des polysaccharides sont connus dans l'état de la technique et peuvent être effectués par l'utilisation d'agents bifonctionnels capables de réagir avec les fonctions hydroxyles des sucres, il s'agit notamment de l'épichlorhydrine, l'épibromhydrine, les bis-époxydes, les anhydrides mixtes de di-acides carboxyliques ou l'oxychlorure de phosphore (POCl$_3$).

Les paramètres particuliers de la mise en oeuvre de ce type de procédé sont connus de l'homme de métier. On obtient généralement un gel. Ce gel doit être broyé mécaniquement afin d'obtenir des particules ayant quelques dizaines de microns de diamètre. Il est ensuite nécessaire pour obtenir des vecteurs selon la présente invention d'effectuer une nouvelle fragmentation, celle-ci pouvant être effectuée par des méthodes ultra-sonores ou par des méthodes telles que l'extrusion à la presse de French.

Pour les vecteurs particulaires selon la présente invention, on préfère utiliser des noyaux ayant une taille de 10 nm à 5 μm, bien que dans certains cas, des particules ayant des tailles supérieures ou inférieures puissent être éventuellement utilisées, les particules préférées sont des particules ayant entre 20 et 70 nm et de préférence aux alentours de 50 nm.

Les propriétés des noyaux polysaccharidiques hydrophiles peuvent être modifiées en substituant les sucres du noyau par des fonctions ioniques, acides ou basiques. Cette substitution qui a lieu d'une manière homogène à l'intérieur et à l'extérieur du noyau peut être introduite avant, pendant ou après la réticulation. Ces propriétés ioniques sont particulièrement intéressantes dans le cas de l'encapsulation de composés eux-mêmes ioniques mais de charge opposée à celle du noyau.

Bien entendu, dans certains cas, on pourra être amené lorsque l'on souhaite avoir des gammes de dimension homogène, à effectuer une purification en fonction des diamètres des particules obtenues, par toute méthode appropriée, notamment par centrifugation différentielle.

La première couche ou couronne est obtenue de préférence par couplage chimique à partir des fonctions hydroxy ou des fonctions hydroxy dérivées du noyau en utilisant des réactifs lipidiques, c'est-à-dire notamment des stérols, des acides gras, des amines grasses, des phospholipides, des acides aminés hydrophobes ou des alkoxyéthers.

Ce type de greffage est connu dans l'état de la technique et consiste à utiliser des réactions de modifications des hydroxy des sucres dans un milieu aprotique non-solvant des polysaccharides tels que le dichlorométhane, l'hexane, le toluène, et le couplage du radical hydrophobe du composé lipidique correspondant.

Au niveau de la synthèse de la première couche lipidique, si les réactions de greffage sont effectivement connues de l'homme de l'art, le procédé selon l'invention apporte cependant une modification intéressante dans un mode de mise en oeuvre préféré en effectuant la réaction dans un milieu aprotique non solvant aprotique des polysaccharides comme le dichlorométhane, l'hexane ou le toluène. Ceci permet, en effet, de réaliser une dérivation superficielle et non dans la masse du noyau comme ce serait le cas avec un solvant des polysaccharides comme la pyridine. Cette modification est très importante car elle permet, d'une part, de conserver un noyau central hydrophile et, d'autre part, de maintenir la structure particulaire du noyau. Cette structure est obtenue en effet grâce à la réaction de réticulation entre les fonctions hydroxyles des sucres mais également grâce à la présence de liaisons hydrogènes entre les sucres qui ne sont pas pontés par l'agent de réticulation. Ces liaisons sont détruites lorsqu'on réalise une dérivation dans la masse en utilisant un solvant des polysaccharides comme la pyridine et on a montré que l'on perdait du même coup la structure particulaire (on obtient alors une structure en fibre du type acétate de cellulose).

Pour favoriser l'encapsulation de composés ioniques au niveau de la première couche lipidique, il a été trouvé qu'il était possible de réaliser des couronnes lipidiques possédant un caractère ionique sous jacent. Ce caractère ionique peut être apporté par une réaction régiosélective du noyau polysaccharidique avec des composés porteurs d'une fonction ionique positive ou négative et d'une autre fonction capable de former une liaison covalente avec les fonctions hydroxy des sucres du noyau polysaccharidique. C'est le cas par exemple des anhydrides internes de diacide carboxylique tels que l'anhydride succinique qui réagissent avec une fonction OH portée par un alcool ou un sucre pour donner un mono ester de l'acide succinique possédant une fonction carboxylique libre. La réaction de dérivation avec les composés ioniques peut être effectuée préalablement à la réaction d'acylation avec les acides gras ou en même temps. On a trouvé que la présence de groupes ioniques à la surface du noyau polysaccharidique permettait le greffage parallèle d'acide gras. Cependant, pour conserver le caractère hydrophobe nécessaire à l'établissement de la couche phospholipidique, on a trouvé que ce caractère ionique doit rester minoritaire (<40 % molaire pour l'acide succinique) et que les acides gras utilisés doivent être suffisamment long ( > C14). Le greffage est réalisé commodément en effectuant la

EP 0 344 040 B1

réaction de dérivation dans un milieu aprotique non solvant des polysaccharides comme mentionné précédemment pour assurer la régiosélectivité mais en utilisant un mélange de réactifs l'un lipidique, l'autre ionique.

Enfin le feuillet lipidique externe peut être réalisé avec des phospholipides ou avec n'importe quel tensioactif ionique ou non ionique capable de s'organiser en micelles comme cela a été dit précédemment. Ce feuillet externe est obtenu de préférence par des méthodes analogues à celles qui servent à la préparation des liposomes, c'est-à-dire injection d'éther ou d'éthanol, la dialyse de détergents, la préparation en phase inverse ou la méthode du ballon qui est la plus connue.

Ces véhicules particulaires sont plus particulièrement destinés à véhiculer dans l'une des couches ou noyaux une molécule à activité biologique.

Parmi ces molécules à activité biologique, il faut citer
– les antibiotiques et les anti-viraux,
– les protéines, les protéoglycanes, les peptides,
– les polysaccharides, les lipopolysaccharides,
– les anticorps,
– les insecticides et fongicides,
– les composés agissant sur le système cardio-vasculaire,
– les anti-cancéreux,
– les anti-malariques,
– les anti-asthmatiques.

Sur le choix de la molécule transportée, il n'y a, a priori, pas de limitations. Pratiquement toutes les molécules possédant une activité biologique peuvent être encapsulées. On peut ajouter bien d'autres composés, par exemple les anti-inflammatoires, les anesthésiques, les contraceptifs, les peptides, les antiparasitaires, les vitamines, les prostaglandines, les neuroleptiques, les antidépresseurs, etc.

Dans certains cas il est possible d'utiliser des vecteurs particulaires selon l'invention uniquement marqués, notamment par des méthodes radioactives ou par des méthodes de type fluorescence ou détection RMN, ceci afin de permettre une imagerie et/ou un diagnostic.

Les principes actifs peuvent être insérés soit à l'intérieur du noyau polysaccharidique, soit dans la couronne lipidique soit dans le feuillet phospholipidique externe. Cette insertion peut être réalisée soit spontanément par l'intermédiaire de liaisons hydrophobes, hydrogènes ou ioniques soit après greffage chimique d'un ligand approprié. Dans ce dernier cas, les molécules peuvent être localisées à l'extérieur de la particule et lui être liées par un ligand hydrophobe. On distingue ainsi quatre zones spatialement définies. C'est cette partition en zones spatiales qui a fait adopter la terminologie Supra Moléculaire.

L'invention concerne enfin les compositions pharmaceutiques ou de diagnostic ou d'imagerie comportant un vecteur particulaire selon l'invention.

Dans les exemples ci-après, on décrira le chargement de différents produits en fonction de leurs caractéristiques et notamment :
– d'un produit hydrophile de petite taille, un antibiotique de la famille des aminoglycosides, la butirosine,
– d'un produit amphiphile, le propranolol, localisé dans la couronne lipidique interne possédant un caractère acide,
– du même propranolol mais localisé cette fois dans le feuillet phospholipidique externe,
– d'un composé très hydrophobe, un antibiotique insecticide, la deltaméthrine, localisé dans la couche lipidique interne,
– d'une protéine membranaire le cytochrome C,
– d'un composé hydrophile de grande taille mais possédant un reste lipidique, le lipopolysaccharide de Salmonella minnesota formé S,
– d'une protéine enzymatique modifiée par des chaines d'acide gras,
– d'un anticorps monoclonal modifié par des chaines d'acide gras,
– d'un antibiotique hydrophobe, l'érythromycine.
– d'une protéine enzymatique native, la péroxydase ancrée à la surface des BVSM,
– de la même péroxydase encapsulée à l'intérieur d'un noyau polysaccharidique acide.

Les véhicules particulaires selon la présente invention peuvent être stérilisés notamment par filtration ou bien par autoclavage à 120°C pendant 20 min par exemple. Ils peuvent être congelés sans précautions particulières. La lyophilisation peut être réalisée en présence d'un additif de lyophilisation comme le maltose, bien que cela ne soit pas indispensable, afin de permettre la réhydratation. Ils peuvent aussi être atomisés. La présence d'un sucre ou d'un polysaccharide dans la suspension à atomiser permet d'enrober les particules avec une pellicule osidique qui contribue à éviter les phénomènes d'agrégation entre les particules et à permettre une meilleure remise en suspension dans l'eau. Les exemples ci-après permettront de mettre en évidence de façon plus claire certains des procédés mis en oeuvre afin d'insérer les produits dans les différentes couches

4

des vecteurs particulaires en cause.

Outre leur degré de variabilité important, les BVSM présentent des caractéristiques physico-chimiques intéressantes.

Parmi les nombreux avantages de ce type de nanoparticules, on peut citer notamment :

– le type de construction modulaire en multicouches autorisant une adaptation au produit à transporter, à son taux d'encapsulation requis ainsi qu'à la cible choisie,

– la possibilité de modification de l'état de surface de la particule pour favoriser sa réactivité ou son adsorption intestinale et cutanée,

– de façon analogue aux liposomes, le feuillet externe phospholipidiques des BVSM présente une similitude avec la membrane cellulaire et est capable d'interagir avec cette dernière,

– une très grande stabilité chimique due à la structure polymérique du noyau polysaccharidique,

– une entière biodégradabilité et biométabolisation,

– une capacité d'encapsulation importante de produit actifs de nature chimique variée,

– une taille définie et la possibilité d'obtenir de façon homogène des très petites tailles (50 nm) permettant une régulation de la diffusion dans les systèmes et tissus biologiques,

– la possibilité d'industrialiser le procédé de fabrication.

Les exemples ci-après permettront de mieux mettre en évidence d'autres caractéristiques et avantages de la présente invention.

## EXEMPLE 1 : PREPARATION DES NOYAUX DE POLYSACCHARIDE RETICULE

Dans un réacteur de 2 litres muni d'un piège à soude, on mélange 150 g de dextrane de poids moléculaire de 229 000 avec 150 ml d'une solution de soude 1 M. Lorsque la solution de dextrane est homogène, on verse 12 ml d'epichlorhydrine, tout en agitant vigoureusement. Le mélange réactionnel est chauffé à 80 °C à l'aide d'un bain-marie. L'agitation est arrêtée après l'ajout de l'epichlorhydrine tandis que la température est maintenue à 80°C pendant 10 heures.

On obtient un gel un peu élastique et cassant. Ce gel est mis en suspension dans 1.850 l d'eau puis broyé mécaniquement avec un appareil à hélice (type Waring blender) afin d'obtenir des particules de quelques dizaines de microns de diamètre.

On effectue ensuite une ultra fragmentation du dextrane reticule pour obtenir les nanoparticules de 50 nm. La fragmentation peut être effectuée par toute méthode suffisament énergétique pour permettre un nanobroyage. Les deux méthodes suivantes sont données à titre d'exemple.

### a) Ultra fragmentation par ultra-sons

La suspension brute précédement obtenue est soniquée à l'aide d'une sonde à ultra-sons (puissance 250 watts) pendant 30 mn. On obtient un mélange hétérogène contenant 20 % de particules ayant un diamètre inférieur à 100 nm.

### b) Fragmentation par la presse de French

La suspension brute précédemment obtenue est extrudée à l'aide d'une presse de French sous une pression réelle de 140 MPascal. Cette opération est répétée 3 fois. La proportion de particules ayant un diamètre supérieur à 100 nm est alors inférieure à 5 %. Le diamètre moyen déterminé par microscopie électronique est de 50 nm.

Les nanoparticules sont purifiées par centrifugation différentielle entre 1000 g et 6000 g (15 mn). Les particules sédimentant en dessous de 2000 g ont une taille supérieure à 100 nm tandis que celles sédimentant entre 2000 et 5000 g ont une taille comprise entre 10 et 100 nm. Ces valeurs sont variables et dépendent des conditions de réticulation et de la nature de polysaccharides employés.

La taille des particules est mesurée en microscopie électronique (grossissement 20.000 et 100.000 fois) et au nanosizer Coulter. Les particules se présentent sous la forme de sphéroïdes relativement réguliers.

Le polysaccharide de départ est retrouvé à 90 % sous forme réticulée insoluble tandis que 10 % est retrouvé sous forme de polysaccharide soluble. Le dosage est effectué avec la méthode de l'anthrone. La déshydratation des particules de polysaccharides réticulés est réalisée à l'aide d'un atomiseur Büchi 190. La concentration des particules est de 5 %, la température de l'air de séchage est de 200°C.

Préparation de noyaux de polysaccharides réticulés acides

50 g de noyaux polysaccharidiques obtenus précédemment par polymérisation et fragmentation sont dispersés dans 1 l d'eau distillée et le pH est ajusté à 8 avec NaOH. On ajoute 20 g d'anhydride succinique et la suspension est agitée pendant 2 heures à température ambiante. Les nanoparticules modifiées sont alors centrifugées et lavées pour éliminer les produits de réaction solubles et les sels. Cette réaction conduit à la formation de monoester de l'acide succinique.

Préparation de noyaux polysaccharidiques réticulés basiques

50 g de noyaux polysaccharidiques sont dispersés comme précédemment dans 1 l d'eau distillée. Le pH est ajusté à 8 et on ajoute 20 g de chlorure de glycidyltriméthylammonium. La suspension est agitée 24 heures à température ambiante. Les nanoparticules modifiées sont centrifugées et lavées pour éliminer les produits de réaction solubles et les sels. Cette réaction conduit au greffage sur les sucres de groupes triméthylamino-1, ol-2 propyloxy-1.

Le taux de substitution est d'environ 0,5 équivalent par sucre, déterminé par titration. Par suite de la répulsion existant entre les fonctions ioniques de même charge, les substituants ioniques sont répartis de façon homogène dans le volume de la particule.

EXEMPLE 2 : PREPARATION DE LA COURONNE LIPIDIQUE AVEC UN CARACTERE LIPIDIQUE SELON L'EXEMPLE 1

A 100 g de particules polysaccharidiques atomisées de diamètre moyen 50 nm mis en suspension dans 1 l de dichlorométhane, sont ajoutés 75 g de diméthylamminopyridine et 100 g de chlorure d'acide octanoïque. L'ensemble est laissé sous agitation à l'abri de l'air, à 37°C pendant 20 h. La récupération et le lavage des particules après réaction est réalisé par évaporation du dichlorométhane suivi de trois lavages au méthanol. Entre chaque lavage, les particules sont récupérées par centrifugation (20 mn à 5000 rpm).

L'étude de la cinétique de la réaction permet, en fonction de la stoechiométrie des composants, du solvant utilisé et de la température choisie d'obtenir une couche lipidique plus ou moins dense et établie plus ou moins profondément à l'intérieur de la particule.

Exemple de variation du taux de greffage d'acide gras en fonction des conditions opératoires pour des particules de diamètre 50 nm.

| Rapport poids de particules/ poids de chlorure d'acide octanoïque | Température (°C) | Durée de réaction (heure) | Taux de greffage (% en poids) |
|---|---|---|---|
| 1 | 37 | 18 | 5 |
| 5 | 37 | 18 | 0,6 |
| 0,5 | 37 | 5 | 1,5 |

Les acides gras greffés sur le noyau polysaccharide ont été dosés par la méthode de LAUWERYS.

EXEMPLE 3 : PREPARATION DE LA COURONNE LIPIDIQUE AVEC UN CARACTERE IONIQUE

Caractère acide :

On modifie le protocole précédent en utilisant un mélange de chlorure d'acide stéarique (C18) et d'anhydride succinique. A 100 g de particules polysaccharidiques atomisées de 50 nm mis en suspension dans 1 l de dichlorométhane sont ajoutés 75 g de diméthylaminopyridine et 160 g de chlorure d'acide stéarique (0,8 équivalent) et 12 g d'anhydride succinique ( 0,2 équivalent). Le rapport molaire C18/anhydride succinique peut être varié et on obtient le taux de greffage suivant :

| Rapport molaire C18/A.succinique | Température °C | durée (heure) | taux de greffage A.G. % en poids |
|---|---|---|---|
| 1 / 0 | 37 | 48 | 10 |
| 0,9/ 0,1 | 37 | 48 | 9,2 |
| 0,75 0,25 | 37 | 48 | 8 |
| 0,6 0,4 | 37 | 48 | 7 |

Caractère basique :!

Caractère basique :!

Un mode opératoire analogue est utilisé avec un mélange chlorure d'acide/chlorure de glycidyltriméthylammonium.

EXEMPLE 4 : PREPARATION DU FEUILLET LIPIDIQUE EXTERNE

A 150 g de particules (30-100 nm) acylées par l'acide octanoïque (5 % d'acide gras) sont ajoutés 30 g de phospholipides (1/3 phosphatidylethanolamine, 1/3 P. choline, 1/3 P. inositol) dans 1,5 l de chloroforme. L'ensemble mis dans un ballon de 5 l est amené à sec sous pression réduite au rotavapor puis, après addition de 2 l d'eau distillée, est placé à 40°C pendant 30 mn en agitant toutes les 10 mn.

On peut utiliser une variante de cette méthode en mélangeant les noyaux acyles et les phospholipides à l'état de poudre et en ajoutant ensuite progressivement la quantité d'eau nécessaire tout en agitant. Cette variante est naturellement plus adaptée à la préparation de quantités importantes de BVSM.

Les BVSM sont ensuite obtenus sous forme d'une suspension homogène par sonication (1 H 30 au bain) ou par extrusion à la presse de French. Les liposomes (SUV) ayant pu se former au cours de l'opération par suite de la présence d'un excès de phospholipides sont éliminés par une centrifugation (15 000 rpm, 30 min), les BVSM ayant sédimentés sont récupérés.

Les BVSM remis en suspension par dispersion quelques minutes au bain à ultra sons se présentent sous la forme d'une solution laiteuse. Cette suspension est stable mais finit par décanter après plusieurs jours, une simple agitation peu énergique suffit alors pour re-obtenir la suspension de départ

Le dosage des phospholipides est effectué par le dosage du phosphore par la méthode d'AMES et DUBIN. Dans le cas des noyaux présentant un taux de greffage d'acide octanoïque de 5 % la quantité de phospholipides associée aux BVSM est de 15 %.

EXEMPLE 5 : STERILISATION DES BVSM DE 50 NM PAR FILTRATION

La stérilisation des BVSM de diamètre inférieure à 100 nm est obtenue par filtration sur membranes d'acétate de cellulose ou de polycarbonate (pores de 0,22 µm de diamètre). Cette filtration nécessite l'emploi d'une pression élevée mais n'entraine pas de phénomènes de colmatage.

Deux filtrations successives sont nécessaires pour obtenir une stérilisation complète. Après filtration, la suspension obtenue est remarquablement homogène et présente une tendance à sédimenter extrêmement faible (plusieurs semaines) qui doit traduire l'état très dispersé de la suspension provoqué par l'effet d'extrusion de la membrane.

Les BVSM ainsi obtenus peuvent être autoclavés sans précautions particulières à 120°C pendant 20 minutes. Après autoclavage, les suspensions de BVSM apparaissent inchangées.

Les BVSM peuvent être également congelés sans précautions particulières. Après décongélation, la suspension obtenue est identique à celle de départ. La lyophilisation directe des BVSM peut être réalisée mais l'adjonction d'un additif de lyophilisation comme le maltose à 5 % permet d'obtenir de meilleurs résultats et une suspension parfaite après lyophilisation et réhydratation (voir paragraphe AED).

EXEMPLE 6 : ETUDE DES BVSM

L'observation en microscopie électronique des BVSM après coloration négative (grossissement 100.000 fois) indique que ces derniers présentent une forme sphéroïde plus ou moins régulière de diamètre moyen de 40 nm.

7

Les particules sont observées à l'état dispersé sans présence d'aggrégats. La présence d'une auréole autour des particules de BVSM et absente sur les noyaux polysaccharidiques seuls est révélatrice de la présence du feuillet externe phospholipidique.

L'étude d'analyse enthalpique différentielle a été réalisée sur des BVSM entourés de dipalmitoylphosphatidylcholine (DPPC) et par comparaison avec des liposomes de DPPC. On observe dans ce dernier cas une température de transition de 40°C qui est en accord avec les données de la littérature. Des températures de transition analogues sont observées pour les BVSM de grandes tailles (20-80 um) et quel que soit l'acide gras présent dans la couronne lipidique : C8-40,9°C, C12-40,2°C, C16-40,7°C. Dans le cas des BVSM de 50 nm, on n'observe une transition que pour les BVSM acylés avec l'acide octanoïque (40°C). Ces résultats indiquent que le feuillet lipidique externe présente bien une architecture organisée de type liposomiale. Cette organisation est toutefois dépendante de la nature des acides gras constituants la couronne lipidique et de la taille des particules.

La température de transition n'est, par ailleurs, pas affectée par la lyophilisation des BVSM.

EXEMPLE 7 : CHARGEMENT DE LA BUTIROSINE DANS LES BVSM

La butirosine (Park Davis) est un antibiotique de la famille des aminoglycosides. C'est une molécule constituée d'un aminocylitol lié par des liaisons glycosidiques à des sucres aminés. Il s'agit donc d'un produit très polaire et ionique, soluble uniquement dans l'eau et les mélanges d'eau et d'alcools inférieurs.

On prépare d'abord des BVSM blancs à partir de 50 g de noyaux polysaccharidiques acylés en C8 (0,6 % d'acide gras) et de 10 g d'asolectine (Fluka) par la méthode du ballon décrite précédemment

Après lavage des BVSM pour éliminer d'éventuels liposomes contaminants, les BVSM sont remis en suspension dans 250 ml d'un mélange méthanol/eau (50:50) contenant 10 g de butirosine sulfate (Parke-Davis). La suspension ainsi obtenue est concentrée sous pression réduite (30°C, 30 mmHg) au moyen d'un évaporateur rotatif. Lorsque la suspension devient pateuse, on rajoute 250 ml du mélange méthanol/eau. L'opération est ainsi répétée trois fois. Après la dernière concentration, le résidu est repris dans 250 ml d'eau puis centrifugé et la butirosine est dosée dans le surnageant par chromatographie liquide haute performance de paire d'ion et par dosage microbiologique sur Bacillus subtilis. Les résultats indiquent la présence de 3 g de butirosine dans le surnageant soit, par différence, 7 g dans le culot contenant les BVSM. On obtient ainsi un taux de charge de 14 % par rapport au poids de noyaux acylés et un rendement d'encapsulation de 70 %. Après lavage de la butirosine libre par centrifugations successives, l'analyse du surnageant des suspensions contenant les BVSM ne permet pas de mettre en évidence la présence de butirosine. Cette dernière est donc encapsulée de manière stable à l'intérieur des BVSM.

La butirosine encapsulée dans les BVSM n'est de plus pas active sur Bacillus subtilis en boîte de Petri.

L'ensemble de ces résultats démontre que la butirosine est localisée à l'intérieur des particules, dans le noyau polysaccharidique.

Des résultats analogues ont été obtenus avec d'autres aminoglycosides notamment la gentamicine.

EXEMPLE 8 : CHARGEMENT DU PROPRANOLOL DANS DES BVSM

a) Chargement dans la couronne lipidique interne à caractère acide

Le propranolol (ICI Pharma) est un béta-bloquant qui possède une structure amphiphile avec une partie lipophile aromatique et une partie ionique basique due à la présence d'une amine secondaire.

On réalise une suspension avec 50 g de noyaux acylés par un mélange chlorure d'acide palmitique, anhydride succinique (75/25) comme décrit précédemment. Les noyaux sont dispersés dans 500 ml d'une solution méthanol/eau (50:50). On ajoute à cette suspension 10 g de propranolol base, on évapore le solvant sous pression réduite (40°C, 30 mm Hg) avec un évaporateur rotatif. Après évaporation complète, le résidu est repris par 200 ml du même mélange méthanol/eau et évaporé à nouveau. Après trois cycles d'évaporation, on ajoute au résidu 10 g de phospholipide (lécithine) sous forme de poudre. On ajoute alors 500 ml d'eau par petites fractions et en agitant régulièrement. La suspension est ensuite homogénéisée à l'aide d'abord d'un appareil type Waring blender puis en soniquant ou en extrudant à la presse de French. La suspension est centrifugée et le propranolol est dosé dans le surnageant par la mesure de l'absorption à 290 nm. On trouve une quantité de 2 g de propranolol dans le surnageant soit par différence 8 g dans les BVSM. Le rendement d'encapsulation est donc de 80 % avec un taux de charge de charge de 16 %.

b) Chargement du propranolol dans le feuillet lipidique externe

On procède ici comme dans le cas de la butirosine. On prépare d'abord les BVSM blancs (C8) puis on ajoute le propranolol dans une solution méthanol/eau (50:50) et on effectue plusieurs cycles de concentration (sans aller à sec toutefois). Le résidu est ensuite resuspendu dans de l'eau, homogénéisé, centrifugé (5000 g, 10 mn) et le propranolol est dosé par spectrométrie UV à 290 nm dans le surnageant. Avec les mêmes quantités que précédemment, on trouve un rendement d'encapsulation de 30 % et un taux de charge de 6 %, soit des valeurs inférieures de moitié aux valeurs précédentes.

EXEMPLE 9 : CHARGEMENT DE LA DELTAMETHRINE DANS LES BVSM

La deltaméthrine (Roussel Uclaf) est un insecticide de la famille des pyréthrinoïdes. C'est un composé extrêmement hydrophobe, soluble seulement dans les solvants organiques.

Le chargement est réalisé à partir de 50 g de noyaux C16 (8 % d'acide gras greffés). Ces noyaux sont mis en suspension dans 500 ml d'éthanol contenant 5 g de deltamethrine. Le solvant est évaporé sous pression réduite (30°C, 30 mm Hg) à l'aide d'un évaporateur rotatif. Le résidu est repris dans 300 ml d'éthanol et évaporé à nouveau. Après trois cycles d'évaporation, on ajoute 10 g de phospholipides (asolectine) au résidu sec et on ajoute 500 ml d'eau par petites fractions en agitant constamment. La suspension est ensuite homogénéisée au mixeur Waring et à la presse de French puis centrifugée. Etant donné l'insolubilité de la deltamethrine dans les solutions aqueuses, le dosage ne s'effectue pas sur le surnageant mais après extraction de la deltaméthrine des BVSM. Après plusieurs lavages (eau et mélange méthanol/eau), la suspension est centrifugée (5000 g, 10 mn). Le surnageant est éliminé et le culot contenant les BVSM est remis en suspension dans l'éthanol absolu (500 ml)). Après agitation pendant 30 mn, la suspension est centrifugée (3000 g, 5 mn), le surnageant est récupéré et le culot est remis en suspension dans 500 ml d'éthanol absolu. Après trois extractions, les surnageants sont rassemblés et la deltamethrine est dosée par mesure de la densité optique à 278 nm.

On trouve 5 g de deltamethrine, soit un rendement d'encapsulation de 100 % et un taux de charge de 10 %. La valeur très élevée du rendement d'encapsulation est certainement due à la grande hydrophobie de la deltaméthrine et à sa non solubilité dans l'eau.

EXEMPLE 10 : CHARGEMENT DU CYTOCHROME C DANS LES BVSM

Le cytochrome C est une protéine membranaire qui doit donc posséder une bonne affinité pour les régions lipidiques : couronne interne et feuillet externe. Pour comparer l'efficacité des différents protocoles, nous avons réalisé l'encapsulation sur les noyaux acylés, sur les BVSM et en conjuguant les deux méthodes.

Les protocoles appliqués sont les mêmes que ceux utilisés précédemment. Les quantités employées sont : 50 g de noyaux C8, 10 g d'asolectine et 10 g de cytochrome C, l'acétonitrile, moins dénaturant pour les protéines, est substitué au méthanol.

Conjugaison des deux méthodes :

Une première encapsulation est réalisée sur les noyaux C8, on prépare ensuite les BVSM par ajout des phospholipides et le traitement habituel. A ce niveau, au lieu de centrifuger les BVSM et de doser le cytochrome dans le surnageant, on rajoute de l'acétonitrile et on refait deux cycles de concentration sous pression réduite (30°C, 30mmHg). Le résidu est alors remis en suspension dans l'eau, homogénéisé, centrifugé et le cytochrome C est dosé dans le surnageant par mesure de la densité optique à 407 nm.

On obtient les résultats suivants :

| Protocole | Rendement d'encapsulation (%) | Taux de charge (%) |
|---|---|---|
| Noyaux acylés | 75 | 15 |
| BVSM 67 | 13,4 | |
| 2 méthodes conjuguées | 82 | 16,4 |

L'utilisation des deux méthodes conjuguées donne les meilleurs résultats.

EXEMPLE 11 : CHARGEMENT DU LIPOPOLYSACCHARIDE (LPS) DE SALMONELLA MINNESOTA (S) DANS LES BVSM

Le LPS est une macromolécule composée d'une partie polysaccharidique (ou O-antigénique) et d'une partie hydrophobe, le lipide A. Il s'agit d'un constituant membranaire des bactéries gram- normalement situé à l'extérieur des bactéries. La localisation choisie pour le LPS est donc le feuillet externe phospholipidique. On reprend ici le protocole utilisé pour la gentamicine et le propranolol en partant des BVSM blancs.

Les BVSM blancs sont préparés comme précédemment, le LPS (L 2137, Sigma) est ajouté aux BVSM en suspension dans un mélange méthanol/eau (30/70). On opère trois cycles de concentration, on resuspend dans l'eau, on homogénise, centrifuge,, et on dose le LPS dans le surnageant par la méthode de Dubois (anthrone). Partant de 50 g de noyaux C8 et de 5 g de LPS, on parvient à encapsuler 4 g de LPS soit un rendement d'encapsulation de 80 % et un taux de charge de 8 %.

EXEMPLE 12 : CHARGEMENT DE L'ALPHA-CHYMOTRYPSINE MODIFIEE PAR DES CHAINES HYDROPHOBES DANS LES BVSM

Préparation de l'alpha-chymotrypsine modifiée :

On ajoute 50 mg de chlorure d'acide palmitique dissous dans 10 ml d'acétone à 100 ml d'un tampon phosphate 10-3 M. 0,145 M NaCl et 1 % de cholate à pH 8. La solution est homogénéisée à l'aide d'un appareil à ultra sons. On ajoute alors l'alpha-chymotrypsine (250 mg) dissous dans 10 ml de tampon. Après deux heures à 4°C, le précipité est séparé par centrifugation (1 heure, 20.000 g) et le détergent est dialysé.

Incorporation de l'alpha-chymotrypsine modifiée dans les BVSM :

On prépare une suspension de BVSM blancs selon la méthode habituelle à partir de 2 g de noyaux C8 et de 400 mg d'asolectine, la solution d'alpha-chymotrypsine modifiée obtenue précédemment est ajoutée à la suspension de BVSM blancs, le mélange est ajusté à 10 % en acétonitrile puis concentré sous pression réduite (1 mmHg, 4°C) au rotavapor. L'opération est répétée 3 fois puis le résidu est remis en suspension dans 50 ml d'eau, homogénéisé et centrifugé.

Le dosage des protéines dans les BVSM par la méthode de Bradford indique une quantité de 200 mg, soit un rendement d'encapsulation de 80 % et un taux de charge de 10 %.

EXEMPLE 13 : CHARGEMENT D'UN ANTICORPS MONOCLONAL MODIFIE PAR DES CHAINES D'ACIDE GRAS DANS LES BVSM

Préparation de l'anticorps modifié :

Un milligramme d'anticomplexe HLA-B2m, SAF1 Class1 (Biosys) purifié est ajouté à 22 µg de N-hydroxysuccinimide ester d'acide palmitique dans 500 µl de tampon PBS contenant 2 % de désoxycholate. Le mélange est incubé à 37°C pendant 12 heures puis chromatographié sur une colonne Séphadex G 75 dans un tampon PBS contenant 0,15 % de désoxycholate pour éliminer l'excès d'acide palmitique. Le pic de volume mort contenant l'immunoglobuline est collecté et dialysé.

Incorporation de l'anticorps dans les BVSM :

On utilise une suspension de BVSM blancs correspondant à 20 mg de noyaux C8 dans 200 µl d'eau. On ajoute la solution d'anticorps obtenue précédemment et on complète à 10 % en acétonitrile. Le mélange est alors concentré sous pression réduite (sans aller à sec) à l'aide d'un appareil type Speed Vac (4°C, 0,01 mmHg). Le mélange est remis en suspension dans 500 µl d'une solution acétonitrile/eau (10:90), puis concentré à nouveau. Après trois cycles de concentration, la suspension est reprise dans 500 µl d'eau, homogénéisée et centrifugée. Le dosage des protéines dans le culot par la méthode de Bradford indique la présence de 1 mg de protéine soit environ 100 % de rendement d'encapsulation et un taux de charge de 5 %.

## EXEMPLE 14 : CHARGEMENT DE L'ERYTHROMYCINE DANS LES BVSM

On réalise une suspension avec 50 g de noyaux acylés en C12 (5 % en poids) dans 500 ml d'un mélange eau/méthanol (50:50). On ajoute à cette suspension 10 g d'érythromycine (Eli Lilly) et on évapore le solvant sous pression réduite (40°C, 30 mmHg) avec un évaporateur rotatif. Après évaporation complète, le résidu est repris par 200 ml du même mélange méthanol/eau et évaporé à nouveau.

Après trois cycles d'évaporation, on ajoute au résidu 10 g de phospholipides (Asolectine) sous forme de poudre. On procède ensuite selon la méthode habituelle. L'érythromycine est dosée dans le surnageant par CLHP sur colonne C18 et détecte en U.V. à 228 nm. Dans le surnageant, on ne trouve pas d'érythromycine, tout le produit a été encapsulé dans les BVSM. Le rendement d'encapsulation est donc de 100 % avec un taux de charge de 20%.

## EXEMPLE 15 : CHARGEMENT DE LA PEROXYDASE A LA SURFACE DES BVSM

La peroxydase (Sigma) est une enzyme d'un poids moléculaire de 40 000 daltons et un point isoélectrique de 7,2. Le but recherché est de parvenir à immobiliser de façon stable l'enzyme sur le support BVSM sans la dénaturer.

BVSM neutres. On utilise des particules polysaccharidiques de taille comprise entre 1 et 3 µm. 50 g de ces particules acylées par le chlorure de l'acide stéarique sont placés dans un ballon de 500 ml et mélangés intimement à 5 g de péroxydase et à 5 g de lécithine hydrogénée. On ajoute alors 250 ml d'eau distillée par petites fractions et en agitant fortement. La suspension obtenue est incubée à 37°C sous forte agitation puis soniquée pendant 1 min au bac à ultra sons. Les BVSM sont centrifugées et la péroxydase non encapsulée est dosée dans le surnageant par la mesure de la densité optique à 402 nm. On trouve 250 mg de péroxydase dans le surnageant soit par différence un taux d'encapsulation de 95 %.

BVSM acides. On utilise des particules polysaccharidiques identiques à celles utilisées précédemment. 50 g de ces particules acylées par un mélange chlorure d'acide stéarique (0,8 équivalent)/anhydride succinique (0,2 équivalent) snt placés comme précédemment dans un ballon de 500 ml et mélangés à 5 g de péroxydase et à 5 g de lécithine hydrogénée. Après le même traitement que précédemment, la suspension est centrifugée et la péroxydase non encapsulée est dosée dans le surnageant par la mesure de la densité optique 402 nm. On trouve 375 g soit un taux d'encapsulation de 92,5 %.

## EXEMPLE 16 : CHARGEMENT DE LA PEROXYDASE DANS LE NOYAU POLYSACCHARIDIQUE ACIDE DES BVSM

10 g de noyaux polysaccharidiques acides sont préparés selon l'exemple 1 par la réaction de noyaux polysaccharidiques avec l'anhydride succinique. Les noyaux ont un diamètre compris entre un et trois microns et possèdent des mailles suffisamment larges pour que des protéines d'un poids moléculaire inférieur à 50 000 daltons puissent pénétrer à l'intérieur des mailles de la particule. Les noyaux sont agités doucement dans 100 ml de tampon acétate pH 5,5 en présence de 5 g de peroxydase pendant 24 heures. Les particules sont ensuite centrifugées et la peroxydase est dosée dans le surnageant par mesure de la densité optique à 402 nm. On trouve 500 mg soit un rendement d'encapsulation de 90 % et un taux d'encapsulation de 45 %. Les particules sont lavées à l'eau distillée, lyophilisées et acylées dans les conditions habituelles avec le chlorure de l'acide stéarique. Les particules acylées sont mélangées avec 1 g de lécithine hydrogénée, on ajoute 150 ml. d'eau distillée par petites fractions en agitant. Une suspension homogène de BVSM est obtenue après agitation 30 min à 37°C. Bien que la réaction d'acylation n'ait lieu qu'à la périphérie des noyaux, il est possible qu'une partie de la péroxydase ait été acétylée au cours de cette réaction.

## EXEMPLE 17 : ETUDE DE LA STABILITE DES PRODUITS ENCAPSULES DANS LES BVSM

Cette étude a été réalisée en détail sur les BVSM chargés au propranolol, comme décrit à l'exemple 7. Des résultats similaires ont également été obtenus avec les autres produits.

La fuite de propranolol a été évaluée par la mesure de la densité optique à 290 nm du surnageant d'une suspension de BVSM acide chargé à 8,4 % en propranolol. Les BVSM (500 mg de noyaux, 42 mg de propranolol) sont mis en suspension dans 20 ml d'eau. L'analyse du surnageant indique la présence de 1,76 mg de propranolol libre soit 4 % du total.

La lyophilisation et l'atomisation ont été réalisées avec l'adjonction de 5 % de maltose (P/V) dans la suspension de BVSM à déshydrater.

| Temps de conservation (jours) en solution à 4°C | 0 | 1 | 2 | 3 | 4 |
|---|---|---|---|---|---|
| D.O. | 0,241 | 0,415 | 0,234 | 0,228 | 0,241 |
| % de propranolol libre | 4 | 6,8 | 3,8 | 3,8 | 4 |

| Congélation | avant | après |
|---|---|---|
| D.O. | 0,241 | 0,295 |
| % de propranolol libre | 4 | 4,8 |

| Lyophilisation | avant | après |
|---|---|---|
| D.O. | 0,241 | 0,247 |
| % de propranolol libre | 4 | 4,1 |

| Atomisation | avant | après |
|---|---|---|
| D.O. | 0,24 | 0,11 |
| % de propanolol libre | 4 | 1,9 |

Dans tous les cas, le taux de fuite est très faible et même diminue avec l'atomisation.

## EXEMPLE 18 : ETUDE DE LA PHARMACOCINETIQUE D'ANTIBIOTIQUES ENCAPSULES

Afin d'évaluer l'influence de l'encapsulation sur la pharmacocinétique de deux antibiotiques : la butirosine selon l'exemple 6, et l'érythromycine selon l'exemple 13, nous avons procédé au dosage plasmatique en fonction du temps de ces deux antibiotiques, après administration sous forme libre et sous forme encapsulée.

Protocole expérimental :

Les injections sont effectuées sur des lapins mâles type "White New-Zealand" d'environ 3 kg, par voie sous-cutanée et intra-veineuse. Les prélèvements sont effectués au niveau de la veine latérale de l'oreille à raison de 200 µl de sang/prélèvement. Le sang est aussitôt centrifugé à 8000 rpm pendant 15 mn. Le sérum est prélevé et conservé à 4°C jusqu'à son utilisation.

Méthode de dosage :

La butirosine, comme l'érythromicine, est dosée par la mesure des zones de lyse (méthode des cylindres d'agar) en milieu gélosé (bioMérieux) ensemencé avec 1000 cfu/ml de Bacillus subtilis et incubé à 37°C pendant 18 heures. Le milieu gélosé bioMérieux consiste en bio-Gelytone 6 g, extrait de levure 3 g, extrait de viande de boeuf 1,5 g et gélose 15 g pour 1 l d'eau distillée. Le milieu est ajusté à pH = 9.

Protocole :

Les échantillons de sérum sont déposés dans les puits réalisés sur le milieu gélosé (30 µl/puit). Des solutions de référence représentant des quantités connues d'antibiotique, sont également déposées sur la boîte.

**BUTIROSINE**

BUTIROSINE

| | | | | | | | |
|---|---|---|---|---|---|---|---|
| µg/ml gamme étalon | 0,5 | 1 | 2 | 5 | 10 | 20 | 50 |
| Diamètre (mm) | 14 | 17 | 19 | 22 | 24 | 26 | 28 |

Injection sous-cutanée

Les quantités injectées sont de 1 ml contenant 9 mg de butirosine sous forme libre ou encapsulée.

| Temps | | | 30 mn | 1h | 2h | 4h | 6h | 12h | 18h |
|---|---|---|---|---|---|---|---|---|---|
| Butirosine libre | Ø mm | | 24 | 23 | 22 | 18 | 13 | 0 | 0 |
| | µg/ml | | 9 | 7 | 5 | 2 | 0,1 | 0 | 0 |
| Butirosine encapsulée | Ø mm | | 0 | 0 | 0 | 16 | 16 | 14 | 15 |
| | µg/ml | | 0 | 0 | 0 | 0,8 | 0,8 | 0,5 | 0,6 |
| BVSM seuls | µg/ml | | 0 | 0 | 0 | 0 | 0 | 0 | 0 |

On constate que la butirosine libre diffuse très rapidement dans le plasma et est éliminée après 6 heures. Au contraire, la butirosine encapsulée apparait dans la circulation beaucoup plus tard (4h) et se maintient à un taux bas mais significatif.

Injection intra-veineuse

On injecte 4 ml d'une solution physiologique contenant 5 mg de butirosine soit libre, soit encapsulée dans 100 mg de BVSM. La solution est préparée à partir de NaCl 8 g/l, KCl 0,2 g/l, $CaC_{12}$ anhydre 0,1 g/l, $MgCl_2$ 0,1 g/l, $Na_2HPO_4$ 3,18 g/l, $KH_2PO_4$ 0,2 g/l, ajustée à pH 7,4.

Les injections sont effectuées lentement dans la veine externe de l'oreille après vasodilatation à l'eau chaude et désinfection à l'éthanol à 70°.

| Temps | 1/2 h | 1 h | 2 h | 3 h | 24 h |
|---|---|---|---|---|---|
| Diamètre d'inhibition (mm) | 24,3 | 23 | 19 | 17 | 0 |
| butirosine libre µg/ml | 11 | 7 | 2 | 1 | 0 |

| Temps | 1/2 h | 1 h | 2 h | 3 h | 24 h |
|---|---|---|---|---|---|
| Diamètre d'inhibition (mm) | 19 | 17 | 17 | 17 | 17 |
| butirosine encapsulée µg/ml | 2 | 1 | 1 | 1 | 1 |

On remarque que la butirosine libre diffuse très rapidement dans le plasma et est éliminée également très rapidement. La butirosine encapsulée commence aussi à diffuser très rapidement mais à un taux plus faible et qui se maintient au cours du temps.

Il semble donc que la métabolisation des BVSM conduisant à la libération de la butirosine soit plus rapide dans le cas de l'injection I.V. que dans l'injection sous cutanée. La libération de l'antibiotique est, dans les deux cas, régulée et stabilisée par l'encapsulation dans les BVSM.

## ERYTHROMYCINE

Les quantités injectées sont de 1 ml contenant 15 mg d'érythromycine libre ou encapsulée. Pour des raisons de solubilité, l'érythromycine libre est dissoute dans un mélange eau-éthanol (85-15).

| µg/ml gamme étalon | 10 | 25 | 100 | 250 | 1 000 |
|---|---|---|---|---|---|
| Diamètre (mm) | 26 | 29 | 31 | 33 | 37 |

| Temps | | 1h | 3h | 6h | 9h | 12h | 24h |
|---|---|---|---|---|---|---|---|
| Erythromycine libre | $\emptyset$ mm | | 37 | 35 | 28 | 27 | 26 |
| | µg/ml | 1 000 | 500 | 25 | 15 | 10 |

| Erythromycine encapsulée | Ø mm | 0 | 32 | 27 | 26 | 26 | 26 |
|---|---|---|---|---|---|---|---|
| | µg/ml | 0 | 250 | 15 | 10 | 10 | 10 |

| BVSM seuls | Ø mm | 0 | 0 | 0 | 0 | 0 | 0 |
|---|---|---|---|---|---|---|---|
| | µg/ml | 0 | 0 | 0 | 0 | 00 | |

On constate que l'érythromycine diffuse relativement lentement dans le plasma. La forme libre permet d'atteindre un taux plasmatique initial plus élevé que la forme encapsulée. Au bout de 24 heures aucune différence n'est plus observée entre les deux formes.

EXEMPLE 19: ETUDE DE L'ACTIVITE ENZYMATIQUE DE LA PEROXYDASE ANCREE A LA SURFACE DES BVSM.

L'activité enzymatique est mesurée par la méthode à l'ortho-phénylène diamine.
Mode opératoire :
On prépare deux solutions de réactifs.
. Solution A : solution d'eau oxygénée à 0,012 % (V/V) de solution commerciale d'eau oxygénée à 33 % dans du tampon citrate (acide citrique/dihydrogéno-phosphate de sodium) à pH 5.
. Solution B : solution d'orthophénylène diamine à 0,04 % (P/V) dans le tampon citrate.
Dans des tubes de 5 ml on verse dans l'ordre :
– la péroxydase témoin ou à doper en solution dans 500 µl de tampon citrate.
– 500 µl de solution A,
– 500 µl de solution B.
On agite au vortex. On incube 30 mn à température ambiante et on mesure la densité optique à 492 nm contre le tampon.

Gamme étalon

| Etalon (µg/ml) | 0,5 | 1 | 1,5 | 2 | 2,5 |
|---|---|---|---|---|---|
| D.O. (492 nm) | 0,135 | 0,380 | 0,405 | 0,550 | 0,675 |

Dosage de l'activité enzymatique de la peroxydase immobilisée sur les BVSM.
Les BVSM contenant la péroxydase sont centifugés, lavés et remis en suspension dans de l'eau. On prélève un aliquot que l'on dilue 10 000 fois et dont on prélève 500 µl pour l'essai enzymatique.

| Echantillon | BVSM neutres | | | BVSM acides | | |
|---|---|---|---|---|---|---|
| | N1 | N2 | N3 | A1 | A2 | A3 |
| Concentration enzyme fixée (µg/ml) | 2,00 | 2,00 | 2,00 | 2,00 | 2,00 | 2,00 |
| D.O. (492 nm) | 0,391 | 0,387 | 0,393 | 0,555 | 0,542 | 0,553 |
| moyenne D.O. | | 0,390 | | | 0,550 | |
| Equivalent enzyme libre | | 1,200µg/ml | | | 2,00 µg/ml | |
| % d'activité | | 60 | | | 100 | |

Il semble donc que l'activité enzymatique de la peroxydase ancrée à la surface des BVSM dépende dans une large mesure de la structure même des BVSM. Compte tenu de la diminution de cinétique réactionnelle qui est inévitablement apportée par l'ancrage de la péroxydase, les résultats obtenus avec les BVSM acides sont particulièrement intéressants.

## Revendications

1. Vecteur particulaire, caractérisé en ce qu'il comporte :
– un noyau hydrophile non liquide ;
– une première couche ou couronne de nature lipidique liée au noyau par des liaisons covalentes ;
– une seconde couche ou feuillet externe de composés amphiphiles liée à la première couche lipidique par des interactions hydrophobes.

2. Vecteur selon la revendication 1, caractérisé en ce que le noyau hydrophile est constitué d'un polymère hydrophile réticulé.

3. Vecteur selon la revendication 2, caractérisé en ce que le polymère hydrophile réticulé est un polysaccharide réticulé.

4. Vecteur selon la revendication 3, caractérisé en ce que le polysaccharide est choisi parmi : le dextrane, l'amidon et la cellulose et leurs dérivés.

5. Vecteur selon l'une des revendications 3 et 4, caractérisé en ce que le dérivé de polysaccharide porte des charges ioniques acide ou basique réparties de façon homogène dans le volume du noyau.

6. Vecteur selon la revendication 5, caractérisé en ce que le dérivé de polysaccharide est obtenu par réaction d'un composé capable de former une liaison covalente avec le facteur OH du polysaccharide et porte en outre une fraction ionique positive ou négative.

7. Vecteur selon l'une des revendications 5 et 6, caractérisé en ce que le composé est l'acide succinique ou ses dérivés.

8. Vecteur selon l'une des revendications 1 à 7, caractérisé en ce que le noyau a une dimension moyenne comprise entre 10 nm et 5 µm.

9. Vecteur selon la revendication 8, caractérisé en ce que le noyau a une dimension moyenne comprise entre 20 et 70 nm.

10. Vecteur selon l'une des revendications 1 à 9, caractérisé en ce que la première couche est constituée par un composé choisi parmi : les stérols, les acides gras, les amines grasses, les phospholipides les acides aminés hydrophobes et les alkoxyéthers, et leurs mélanges.

11. Vecteur selon l'une des revendications 1 à 10, caractérisé en ce que la couche lipidique est couplée chimiquement au noyau par les fonctions hydroxy ou les fonctions dérivés des fonctions hydroxy desdits noyaux.

12. Vecteur selon l'une des revendications 1 à 11, caractérisé en ce que la seconde couche est constituée d'un phospholipide ou d'un agent tensioactif.

13. Vecteur selon l'une des revendications 1 à 12, caractérisé en ce qu'il comporte, en outre, dans l'une des couches ou le noyau, une molécule à activité biologique.

14. Vecteur selon l'une des revendications 1 à 13, caractérisé en ce que la molécule à une activité biologique est choisie parmi :

– les antibiotiques et les anti-viraux,

– les protéines, les protoglycanes, les peptides,

– les polysaccharides, les lipopolysaccharides,

– les anticorps,

– les insecticides et fongicides,

– les composés agissant sur le système cardio-vasculaire,

– les anti-cancéreux,

– les anti-malariques,

– les anti-asthmatiques.

15. Vecteur selon l'une des revendications 1 à 14, caractérisé en ce que le vecteur est marqué.

16. Vecteur selon la revendication 15, caractérisé en ce que le vecteur est marqué radioactivement.

17. Vecteur selon l'une des revendications 1 à 16, caractérisé en ce qu'il est stérilisé.

18. Vecteur selon l'une des revendications 1 à 17, caractérisé en ce qu'il est sous forme lyophilisé ou atomisé avec un agent de lyophilisation.

19. Vecteur selon la revendication 18, caractérisé en ce que l'agent de lyophilisation est un sucre.

20. Procédé de préparation d'un vecteur particulaire selon l'une des revendications 1 à 19, caractérisé en ce que :

a) on prépare un noyau par réticulation d'un polymère hydrophile dans des conditions permettant d'obtenir des particules de l'ordre de 10 nm à 5 $\mu$m,

b) on couple chimiquement sur les fonctions réactives à la surface du noyau des composés lipidiques afin de former la première couche,

c) on introduit enfin des composés amphiphiles en contact hydrophobe avec la première couche afin de créer la seconde couche.

21. Procédé selon la revendication 20, caractérisé en ce que dans l'étape a), les particules sont obtenues par fragmentation.

22. Procédé selon la revendication 20 ou 21, caractérisé en ce que l'étape b) est effectuée dans un milieu aprotique non solvant des polysaccharides.

23. Procédé selon l'une des revendications 20 à 22, caractérisé en ce que l'étape c) est conduite à l'aide d'une méthode de préparation des liposomes.

24. Procédé selon l'une des revendications 20 à 23, caractérisé en ce qu'on introduit une molécule à activité biologique durant l'une des étapes du procédé ou dans le vecteur terminé.

25. Composition à activité biologique, caractérisée en ce qu'elle comporte un vecteur selon l'une des revendications 1 à 19.

26. Composition pharmaceutique caractérisée en ce qu'elle comporte à titre d'agent actif un vecteur particulaire selon l'une des revendications 13 et 14.

27. A titre d'agent de diagnostic, un véhicule particulaire selon l'une des revendications 15 et 16.

**Patentansprüche**

1. Teilchenförmiger Vektor, dadurch gekennzeichnet, daß er umfaßt:

– einen hydrophilen, nicht-flüssigen Kern;

– eine erste Schicht oder Krone, lipider Natur, gebunden an den Kern durch kovalente Bindungen;

– eine zweite Schicht oder äußeres Blatt aus amphiphilen Verbindungen gebunden an die erste Lipidschicht durch hydrophobe Wechselwirkungen.

2. Vektor nach Anspruch 1, dadurch gekennzeichnet, daß der hydrophile Kern aus einem vernetzten hydrophilen Polymer besteht.

3. Vektor nach Anspruch 2, dadurch gekennzeichnet, daß das vernetzte hydrophile Polymer ein vernetztes Polysaccharid ist.

4. Vektor nach Anspruch 3, dadurch gekennzeichnet, daß das Polysaccharid ausgewählt ist aus: Dextran, Stärke und Zellulose und ihren Derivaten.

5. Vektor nach einem der Ansprüche 3 und 4, dadurch gekennzeichnet, daß das Polysaccharid ionische saure oder basische Ladungen, homogen im Volumen des Kerns verteilt, trägt.

6. Vektor nach Anspruch 5, dadurch gekennzeichnet, daß das Polysaccharidderivat erhalten wird durch Reaktion einer zur Bildung einer kovalenten Bindung mit dem OH-Faktor des Polysaccharids geeigneten Verbindung und außerdem eine positive oder negative ionische Fraktion trägt.

7. Vektor nach einem der Ansprüche 5 und 6, dadurch gekennzeichnet, daß die Verbindung Bernsteinsäure oder ihre Derivate ist.

8. Vektor nach einem der Ansprüche 1 bis 7, dadurch gekennzeichnet, daß der Kern eine mittlere Abmessung von 10 nm bis 5 μm hat.

9. Vektor nach Anspruch 8, dadurch gekennzeichnet, daß der Kern eine mittlere Abmessung von 20 bis 70 nm hat.

10. Vektor nach einem der Ansprüche 1 bis 9, dadurch gekennzeichnet, daß die erste Schicht aus einer Verbindung besteht, ausgewählt aus: den Sterinen, den Fettsäuren, den Fettaminen, den Phospholipiden, den hydrophoben Aminosäuren und den Alkoxyethern und ihren Gemischen.

11. Vektor nach einem der Ansprüche 1 bis 10, dadurch gekennzeichnet, daß die Lipidschicht chemisch an den Kern durch Hydroxyfunktionen oder Funktionen, die sich von Hydroxyfunktionen der Kerne ableiten, gekuppelt ist.

12. Vektor nach einem der Ansprüche 1 bis 11, dadurch gekennzeichnet, daß die zweite Schicht aus einem Phospholipid oder einem oberflächenaktiven Mittel besteht.

13. Vektor nach einem der Ansprüche 1 bis 12, dadurch gekennzeichnet, daß er außerdem in einer der Schichten oder dem Kern ein aktives bio logisches Molekül aufweist.

14. Vektor nach einem der Ansprüche 1 bis 13, dadurch gekennzeichnet, daß das Molekül mit biologischer Aktivität ausgewählt ist aus:
– den Antibiotika und den Antivirusmitteln,
– den Proteinen, den Protoglykanen, den Peptiden,
– den Polysacchariden, den Lipopolysacchariden,
– den Antikörpern,
– den Insektiziden und Fungiziden,
– den auf das Kardiovaskulär-System wirkenden Verbindungen,
– den Antikrebsmitteln,
– den Antimalariamitteln,
– den Antiasthmamitteln.

15. Vektor nach einem der Ansprüche 1 bis 14, dadurch gekennzeichnet, daß der Vektor markiert ist.

16. Vektor nach Anspruch 15, dadurch gekennzeichnet, daß der Vektor radioaktiv markiert ist.

17. Vektor nach einem der Ansprüche 1 bis 16, dadurch gekennzeichnet, daß er sterilisiert ist.

18. Vektor nach einem der Ansprüche 1 bis 17, dadurch gekennzeichnet, daß er in lyophilisierter oder atomisierter Form mit einem Lyophilisierungsmittel vorliegt.

19. Vektor nach Anspruch 18, dadurch gekennzeichnet, daß das Lyophilisierungsmittel ein Zucker ist

20. Verfahren zur Herstellung eines teilchenförmigen Vektors nach einem der Ansprüche 1 bis 19, dadurch gekennzeichnet, daß man:
a) einen Kern durch Vernetzen eines hydrophilen Polymers, unter Bedingungen, die die Erzielung von Teilchen in der Größenordnung von 10 nm bis 5 μm erlauben, herstellt,
b) an die reaktiven Funktionen an der Oberfläche des Kerns Lipidver bindungen chemisch kuppelt, um die erste Schicht zu bilden,
c) schließlich amphiphile Verbindungen in hydrophoben Kontakt mit der ersten Schicht einführt, um die zweite Schicht auszubilden.

21. Verfahren nach Anspruch 20, dadurch gekennzeichnet, daß in der Stufe a) die Teilchen durch Fragmentieren erhalten werden.

22. Verfahren nach Anspruch 20 oder 21, dadurch gekennzeichnet, daß die Stufe b) in einem aprotischen Medium, das kein Lösungsmittel für Polysaccharide ist, durchgeführt wird.

23. Verfahren nach einem der Ansprüche 20 bis 22, dadurch gekennzeichnet, daß die Stufe c) nach einer Methode zur Herstellung von Liposomen durchgeführt wird.

24. Verfahren nach einem der Ansprüche 20 bis 23, dadurch gekennzeichnet, daß man ein Molekül mit biologischer Aktivität während einer der Stufen des Verfahrens oder in den fertig gestellten Vektor einführt.

25. Zusammensetzung mit biologischer Aktivität, dadurch gekennzeichnet, daß sie einen Vektor nach einem der Ansprüche 1 bis 19 umfaßt.

26. Pharmazeutische Zusammensetzung, dadurch gekennzeichnet, daß sie als Wirkstoff einen teilchenförmigen Vektor nach einem der Ansprüche 13 und 14 umfaßt.

27. Teilchenförmiges Vehikel nach einem der Ansprüche 15 und 16 als diagnostisches Mittel.

## Claims

1. Particulate carrier, characterized in that it comprises:
– a hydrophilic, non-liquid core;
– a first layer or ring of lipid nature bonded to the core by covalent bonds;
– a second layer or outer shell of amphiphilic compounds bonded to the first lipid layer by hydrophobic interactions.

2. Carrier according to claim 1, characterized in that the hydrophilic core consists of a crosslinked hydrophilic polymer.

3. Carrier according to claim 2, characterized in that the crosslinked hydrophilic polymer is a crosslinked polysaccharide.

4. Carrier according to claim 3, characterized in that the polysaccharide is chosen from: dextran, starch and cellulose and their derivatives.

5. Carrier according to one of claims 3 and 4, characterized in that the polysaccharide derivative carries acid or basic ionic charges distributed in a homogeneous manner in the volume of the core.

6. Carrier according to claim 5, characterized in that the polysaccharide derivative is obtained by reaction of a compound capable of forming a covalent bond with the OH function of the polysaccharide and also carries a positive or negative ionic fraction.

7. Carrier according to one of claims 5 and 6, characterized in that the compound is succinic acid or its derivatives.

8. Carrier according to one of claims 1 to 7, characterized in that the core has an average size of between 10 nm and 5 $\mu$m.

9. Carrier according to claim 8, characterized in that the core has an average size of between 20 and 70 nm.

10. Carrier according to one of claims 1 to 9, characterized in that the first layer consists of a compound chosen from: sterols, fatty acids, fatty amines, phospholipids, hydrophobic amino acids and alkoxy ethers, and their mixtures.

11. Carrier according to one of claims 1 to 10, characterized in that the lipid layer is chemically coupled to the core by the hydroxyl functions or the functions derived from the hydroxyl functions of the said cores.

12. Carrier according to one of claims 1 to 11, characterized in that the second layer consists of a phospholipid or of a surfactant.

13. Carrier according to one of claims 1 to 12, characterized in that it also comprises, in one of the layers or the core, a molecule having biological activity.

14. Carrier according to one of claims 1 to 13, characterized in that the molecule having a biological activity is chosen from:
– antibiotics and antiviral agents,
– proteins, proteoglycans and peptides,
– polysaccharides and lipopolysaccharides,
– antibodies,
– insecticides and fungicides,
– compounds acting on the cardiovascular system,
– anti-cancer agents,
– anti-malarial agents and
– anti-asthmatic agents.

15. Carrier according to one of claims 1 to 14, characterized in that the carrier is labeled.

16. Carrier according to claim 15, characterized in that the carrier is labeled radioactively.

17. Carrier according to one of claims 1 to 16, characterized in that it is sterilized.

18. Carrier according to one of claims 1 to 17, characterized in that it is in the lyophilized form or atomized with a lyophilization agent.

19. Carrier according to claim 18, characterized in that the lyophilization agent is a sugar.

20. Process for the preparation of a particulate carrier according to one of claims 1 to 19, characterized in that:
a) a core is prepared by crosslinking a hydrophilic polymer under conditions enabling particles of the order of 10 nm to 5 $\mu$m to be obtained,
b) lipid compounds are chemically coupled to the reactive functions at the surface of the core in order to form the first layer, and
c) finally amphiphilic compounds are introduced in hydrophobic contact with the first layer in order to create the second layer.

21. Process according to claim 20, characterized in that in step a) the particles are obtained by fragmentation.

22. Process according to claim 20 or 21, characterized in that step b) is carried out in an aprotic medium which is a non-solvent for polysaccharides.

23. Process according to one of claims 20 to 22, characterized in that step c) is conducted with the aid of a method for the preparation of liposomes.

24. Process according to one of claims 20 to 23, characterized in that a molecule having biological activity is introduced during one of the steps of the process or into the finished carrier.

25. Composition having biological activity, characterized in that it comprises a carrier according to one of claims 1 to 19.

26. Pharmaceutical composition characterized in that it comprises, as active agent, a particulate carrier according to one of claims 13 and 14.

27. A particulate vehicle according to one of claims 15 and 16, as a diagnostic agent.